# EUROPEAN PATENT SPECIFICATION

(11) **EP 4 396 083 B1**
(45) Date of publication and mention of the grant of the patent: **28.10.2026**
(21) Application number: 22865503.1
(22) Date of filing: 31.08.2022
(51) Int. Cl.: B64D 13/06, A61L 9/20, B64D 11/02, E03D 9/04, F24F 8/22, A61L 2/10, A61L 9/12

(54) **AIRBORNE PATHOGEN SANITIZATION SYSTEM**
LUFTGESTÜTZTES PATHOGENDESINFEKTIONSSYSTEM
SYSTÈME D'ASSAINISSEMENT D'AGENTS PATHOGÈNES AÉROGÈNES

(30) Priority: 31.08.2021 US 202117462386
(43) Date of publication of application: 10.07.2024
(73) Proprietor: Safran Cabin, Inc., Huntington Beach, CA 92647 (US)
(72) Inventor: SMITHSON, Tyler, 77550 Moissy-Cramayel (FR); BIRBECK, Tim, 77550 Moissy-Cramayel (FR); TOMASENA, Etienne, 77550 Moissy-Cramayel (FR)
(74) Representative: Casalonga
(86) International application number: PCT/US2022/042203
(87) International publication number: WO 2023/034414

(56) References cited:
- US-A1- 2005 061 656
- US-A1- 2007 053 188
- US-A1- 2019 060 496
- US-A1- 2020 009 286
- US-A1- 2020 009 286
- US-A1- 2020 108 166
- US-A1- 2020 108 166
- US-A1- 2021 128 764
- US-B1- 6 893 610
- US-B2- 10 264 931
- US-B2- 7 279 144

## Description

### FIELD OF THE INVENTION

The present invention relates generally to air sanitizing systems, and more specifically to an air sanitization system for an aircraft lavatory.

### BACKGROUND OF THE INVENTION

Commercial Airline operations are being disrupted by the COVID 19 crisis. Airline passenger trips have reduced significantly causing a financial crisis in the industry. Airlines are interested in developing and deploying technology, products, and solutions that will clean, sanitize, disinfect, or even sterilize the aircraft interior before, during, or after flight. Many solutions are proposed and discussed within the industry and within the general media. These solutions can include wiping surfaces with cleaning liquids, replacing the materials used in high-touch areas with materials that have anti-microbial properties, adding films to high touch surfaces with anti-microbial properties, or using UCV lights as pathogen killing devices. COVID-19 is considered an airborne pathogen, and the present invention is related to the sanitization of airborne pathogens.

When a toilet is flushed, there is a violent air exchange. This event can cause excrement, which may have e-coli or virus present (such as SARS-COV-2) to be dispersed into a lavatory. The dispersion can include very small particles, these particles, which may have pathogens present, can then be present in the lavatory. Cleaning on sterilizing surfaces may not eliminate the presence of airborne pathogens in an enclosed space. There is a need for solutions that not only clean the surfaces of an enclosed space, but also the volume of air within the enclosed space.

Commercial aircraft have confined spaces. One such confined space is a lavatory, another confined space is a galley. Before, during, and after flights the crew and passengers use these confined spaces. Due to the nature of the activities in these confined spaces, there can be smells and odors present that can be unpleasant to the crew and passengers. Passengers often associates smells with cleanliness. Sanitizing air with a UV light, or other means, may not eliminate odors and smells. There is a need to also create a pleasant odor in these confined spaces. High end scent generation is today available in multiple forms. For example, scent marketing is used to enhance customer experience in retail stores, food and beverage stores, casinos and hotels. The aim is typically to put the customer in a positive mood or to otherwise make the customer feel comfortable. The aim can also be to provide a scent that people associated with a clean, fresh, or natural environment. By coupling the sanitization system described herein with a scent machine a total clean environment that is potentially free of many harmful bacteria and viruses can be provided and can therefore provide for a pleasant odor free or odor scent enhanced experience.

Many sanitization systems target the cleaning of surfaces, but not the volume of air. This is a critical concern in an aircraft lavatory since the COVID-19 pandemic began. There is a need for a system that can sanitize airborne pathogens quickly and effectively.

The current state of the art for air sanitization include UV radiators. UV radiators have UV light sources inside of an enclosed housing (typically a cylinder). There are UV reflective coatings applied to the inside of the housing. As air passes through the housing, the air particles are bombarded by UV light, which kills pathogens once a threshold is achieved. US 20201108166 discloses a sanitization system according to the prior art.

The current state of the art for aircraft air filtration is the use of HEPA filters. HEPA filters are very efficient in removing unwanted contaminates and from air, but they greatly reduce the air flow within the system. Also, there is a pressure limit at which the system can effectively operate when using a HEPA filter.

Typically aircraft filtration systems use HEPA filters to filter contaminates and pathogens from the air. HEPA filters greatly restrict airflow within a system. Using a UV air sanitization system would allow the airflow within the system to be significantly increased.

HEPA filters are typically installed in a central location in the aircraft once the air leaves the HEPA filter it may combine with local pathogens. For example, when a passenger sneezes inside a lavatory, the exhaled air may include moisture laden pathogens. There is now a local condition where pathogens are present. Similarly, when a passenger uses a toilet, or a toilet is flushed, non-desirable odors and smell are generated. The central HEPA filter does not prevent this.

### SUMMARY OF THE PREFERRED EMBODIMENTS

The present invention includes a sanitization assembly comprising an air duct, according to claim 1, that can be used to sanitize the air inside of an enclosed space to remove, kill, destroy or eliminate airborne pathogens. The air duct contains UV light sources or emitters located in a pattern around the interior surface of the air and a UV reflective coating on the inside of the duct in order to provide enough energy to kill airborne pathogens in a short amount of time. Because UV is being used to sanitize the air and not a HEPA filter, the airflow within the system can be significantly increased with a fan forcing optimal dwell time pathways. Lastly, a fragrance dispenser can be placed at or near the outlet of the air sanitization system to give the clean air a refreshing scent. As a result, the present invention creates a sanitization system that can quickly kill airborne pathogens that are present within an aircraft lavatory that is safe for occupants, produces a scent that can be desirable for occupants and has low power requirements.

In a preferred embodiment, a UV radiator is located at or near the ceiling of an aircraft lavatory. This can be either near the entry door to the lavatory, next to the mirror or over the toilet in the lavatory. The UV radiator includes a cylindrical duct (or other shape such as a polygon, ovular, etc.). Located on the interior surface of the cylindrical duct are UV light sources. Preferably, the interior of the duct is open with no middle interior items of any kind so that air flow and reflectivity have optimized performance. However, this is not a limitation. The UV light sources can be located in a helix pattern, or other similar pattern to maximize coverage of the UV light waves within the cylinder. The interior surface of the duct may be coated with UV reflective materials. In addition, a fan may be located within the duct. The fan pushes the air through the duct while the UVC light sources bombard the air particles with UV energy. The UV reflective coating increases the amount of UV energy that is absorbed by the pathogens, which destroys them.

In a preferred embodiment, there is an inlet and an outlet to the cylindrical duct. A fragrance dispenser is located at or near the outlet of the duct, which adds a refreshing scent to the sanitized air. By placing the fragrance dispenser in-line with the UV system one can reduce the need of an additional fan. Sensors, controller and other parts can be shared to reduce the overall weight, complexity and costs of the combined system(s).

**In** another preferred embodiment, there is an edge placement of inlet and opposing edge placement of outlet, or a flow redirection surface at the inlet and outlet that force the air to circulate in a helical pattern, increasing dwell time and reducing disinfection time.

**In** another preferred embodiment, the system can be placed in, on or near a galley to have similar benefits to those described herein with respect to a lavatory.

**In** another preferred embodiment, the UV may be located underneath the lavatory mirror. **In** this embodiment, a portion of the cylindrical duct is replaced with a UV transparent lens, which faces toward the sink. The UV transparent lens allows UV light waves to exit the cylinder and contact the surface of the lavatory sink, which sanitizes the surfaces of the sink while also sanitizing the air that is passing through the UV radiator duct. Again, in this embodiment, an air fragrance dispenser is located at the outlet of the UV radiator duct which adds a refreshing scent to the sanitized air.

It will be appreciated that one or more embodiments (a combination of a UV radiator located under the mirror, over the door, and/or near the lavatory ceiling) can be used in the solution.

Using UV light is one method of destroying pathogens. Once a particular amount of UV energy is absorbed by the pathogen, it is destroyed. **In** order to increase the rate at which UV energy is absorbed by pathogens, UV reflective coatings can be applied to the interior surface of an air duct, which increases the amount of energy that is directed at destroying pathogens and not being wasted as heat. With the interior free of objects, the reflection is highly optimized.

It will be appreciated that there are three different functions being addressed by the present invention: clean air, scent air, and clean surfaces, by combination of design elements all three, or two, or one, function can be addressed.

One of the key enablers of the present invention is the UV reflective coating that may be applied to the interior of the air duct and also the locations of the UV light sources on the interior surface of the duct. These enablers greatly increases the amount of energy that is directed at airborne pathogens which decreased the time that it takes for the pathogens to be destroyed. This reflective surface also creates light paths within and outside of the device. The light paths outside of the device are used to clean surfaces. The less time it takes for the pathogens to be destroyed, the greater the air flow within the system can be. This lowers the amount of time needed to sanitize the full volume of lavatory air. The type of reflective surface and the associated percent of reflectivity has an exponential effect on intensity.

By combining the air flow over the LED light sources with airflow over the scent generating device (e.g., a wicking fabric with scent liquid, see U.S. Patent App. 2017/0253338, as an example), with the scent device upstream of the UV device, a single motor driven fan can be used and this air flow can also be used to cool the LEDs.

A plurality of sanitization assemblies can also be part of a sanitization system that are in wireless or wired communication with one another and/or a controller that controls the sanitization assemblies and the various components, such as the fan, the lights, the scent dispenser, etc. (e.g., turn the fan on and off, adjust or changes the speed of the fan, turn the lights on and off, adjust or change the intensity, direction or duration of the lights, dispense scent or sanitizer).

It will be appreciated that LEDs during their operation generate heat, which can cause LED temperatures to rise. LED reliability and life is impacted by temperature. When LEDs are used, the designer of the product has to ensure sufficient cooling methods available, which can include convective, radiative, or conductive paths. The requirement to remove heat from LEDs can cause results that are not desirable to the customers (as they drive weight and complexity). Therefore, it is beneficial for the product design to be able to use cooling paths generated by the surrounding equipment. In a preferred embodiment, the air travelling through the duct portion, that is pushed or delivered from the fan has an air flow that is sufficient or capable of cooling the LEDs. The air movement is created by the same fan used to push the air through the sanitization system.

### BRIEF DESCRIPTION OF THE DRAWINGS

The invention may be more readily understood by referring to the accompanying drawings in which:
FIG. 1 is a perspective view of a lavatory monument assembly including a plurality of sanitizing assemblies therein in accordance with a preferred embodiment of the present invention;
FIG. 2 is perspective view of a portion of the lavatory monument assembly of FIG. 1;
FIG. 3 is a cross-sectional perspective view of an interior/exterior sanitization assembly;
FIG. 4 is a cross-sectional perspective view of an interior sanitization assembly;
FIG. 5A is a cross-sectional elevational view of a sanitization assembly including a plurality of light emitters arranged in a circumferential array;
FIG. 5B is a cross-sectional elevational view of a sanitization assembly including a plurality of light emitters arranged in a circumferential array;
FIG. 5C is a cross-sectional elevational view of a sanitization assembly including a plurality of light emitters arranged in a circumferential array; and
FIG. 5D is a cross-sectional elevational view of a sanitization assembly including a plurality of light emitters arranged in a circumferential array.

Like numerals refer to like parts throughout the several views of the drawings.

### DETAILED DESCRIPTION OF THE PREFERRED EMBODIMENTS

The following description and drawings are illustrative and are not to be construed as limiting. Numerous specific details are described to provide a thorough understanding of the disclosure. However, in certain instances, well-known or conventional details are not described in order to avoid obscuring the description. References to one or an embodiment in the present disclosure can be, but not necessarily are references to the same embodiment; and, such references mean at least one of the embodiments. If a component is not shown in a drawing then this provides support for a negative limitation in the claims stating that that component is "not" present. However, the above statement is not limiting and in another embodiment, the missing component can be included in a claimed embodiment.

Reference in this specification to "one embodiment," "an embodiment," "a preferred embodiment" or any other phrase mentioning the word "embodiment" means that a particular feature, structure, or characteristic described in connection with the embodiment is included in at least one embodiment of the-disclosure and also means that any particular feature, structure, or characteristic described in connection with one embodiment can be included in any embodiment or can be omitted or excluded from any embodiment. Furthermore, any particular feature, structure, or characteristic described herein may be optional.

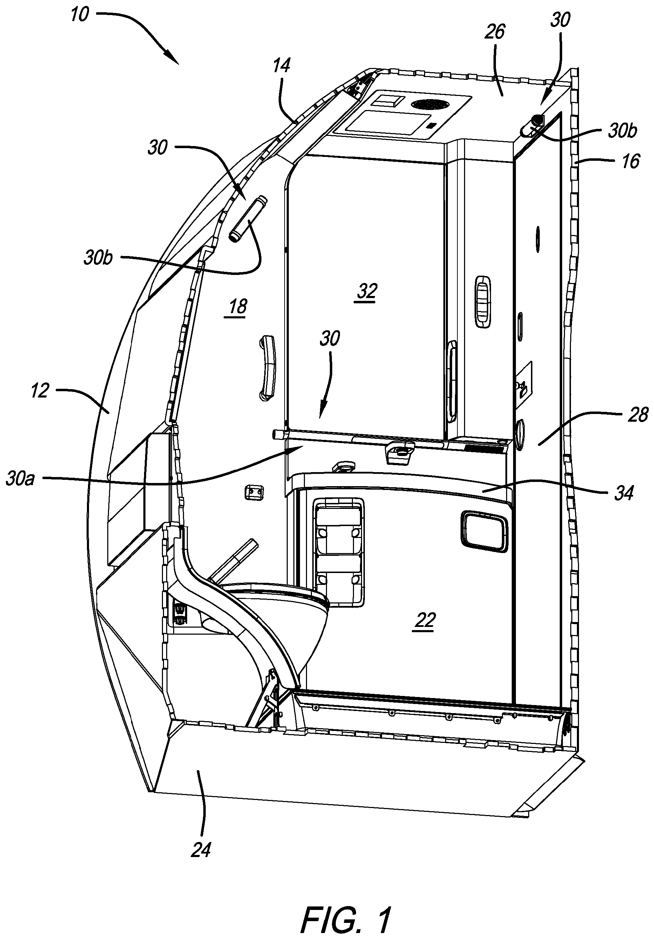

Referring now to the drawings, which are for purposes of illustrating the present invention and not for purposes of limiting the same, FIGS. 1-5D show a vehicle lavatory monument assembly 10 that includes one or more sanitization assembles 30 therein. In a preferred embodiment, the lavatory monument assembly is utilized in an aircraft. However, any type of vehicle, such as a train, bus, etc. is within the scope of the present invention. As shown in FIG. 1, the lavatory monument assembly 10 generally includes an enclosure 12 that includes a plurality of walls (first wall 14, second wall 16, third wall 18 and fourth wall (now shown)) that cooperate to define a lavatory interior 22. The enclosure 12 preferably also includes a floor 24, a ceiling 26 and an entry door 28 that is positioned on one of the plurality of walls. In the figures, the entry door 28 is shown positioned on the second wall 16. However, this is not a limitation and the door can be positioned on any wall. In a preferred embodiment, the lavatory monument assembly 10 includes one or more sanitization assemblies 30 positioned in the lavatory interior 22.

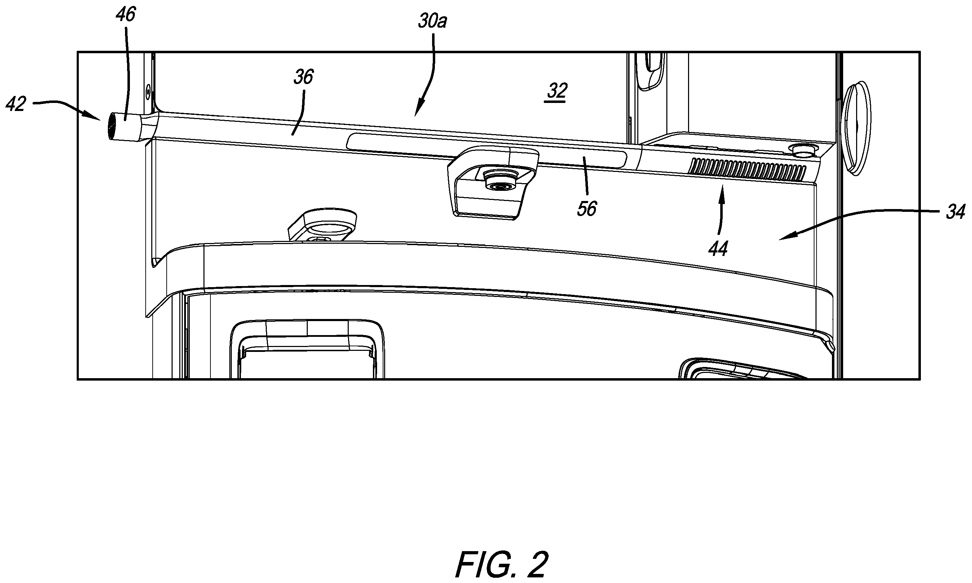

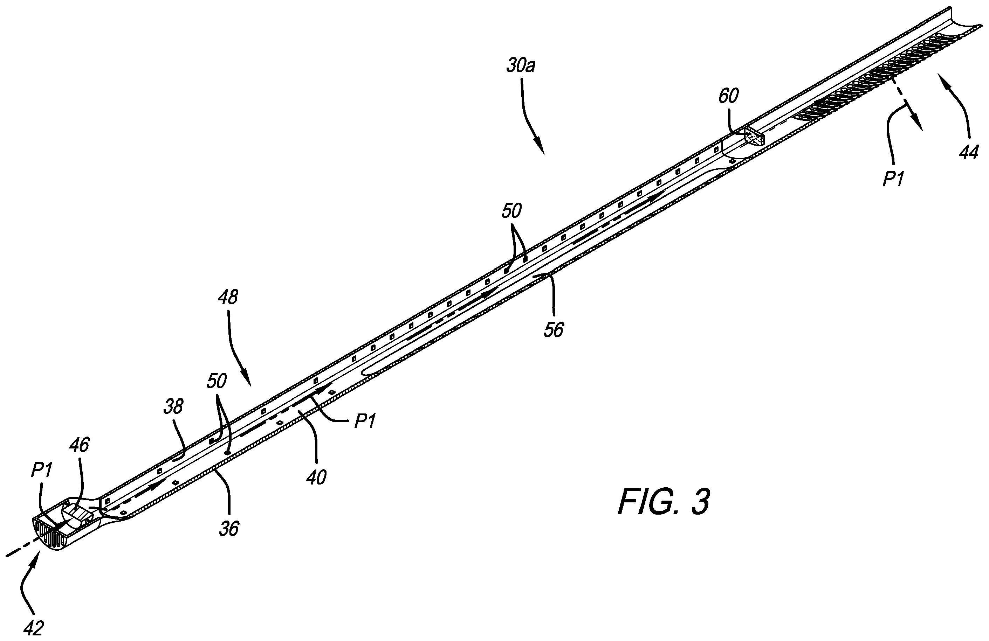

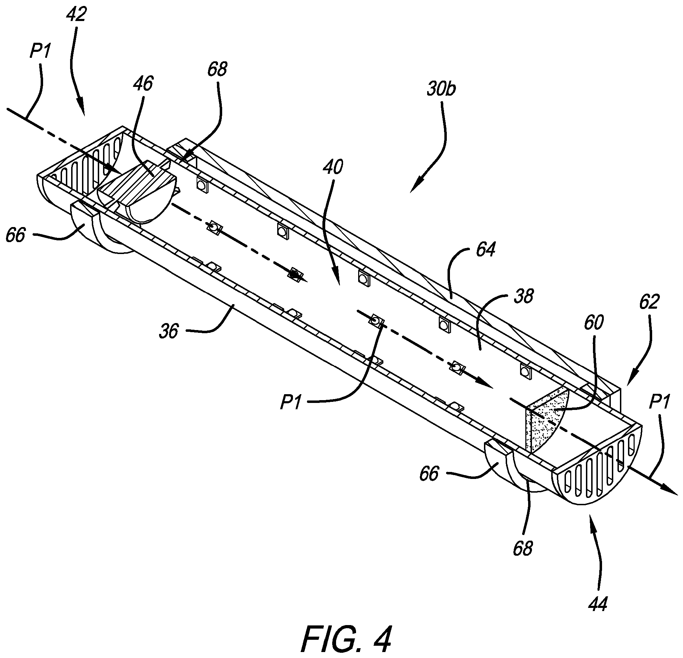

FIG. 1 shows three sanitization assemblies 30 therein, a first sanitization assembly 30 below mirror 32 and above the sink 34, a second sanitization assembly 30 on the third wall next to the mirror 32 and a third sanitization assembly 30 above the door 28. Any number of sanitization assemblies within the lavatory is within the scope of the present invention. FIG. 1 also shows two different types of sanitization assemblies 30. The first type of sanitization assembly 30 that is positioned above the sink (see FIGS. 2 and 3) is also referred to generally herein as an interior/exterior sanitization assembly and is numbered 30a. The second type of sanitization assembly 30 (see FIG. 4) is referred to herein as an interior sanitization assembly and is numbered 30b. Therefore, the sanitization assemblies are labeled/numbered generally as 30 and the interior/exterior sanitization assembly is labeled/numbered specifically as 30a and the interior sanitization assembly is labeled/numbered specifically as 30b.

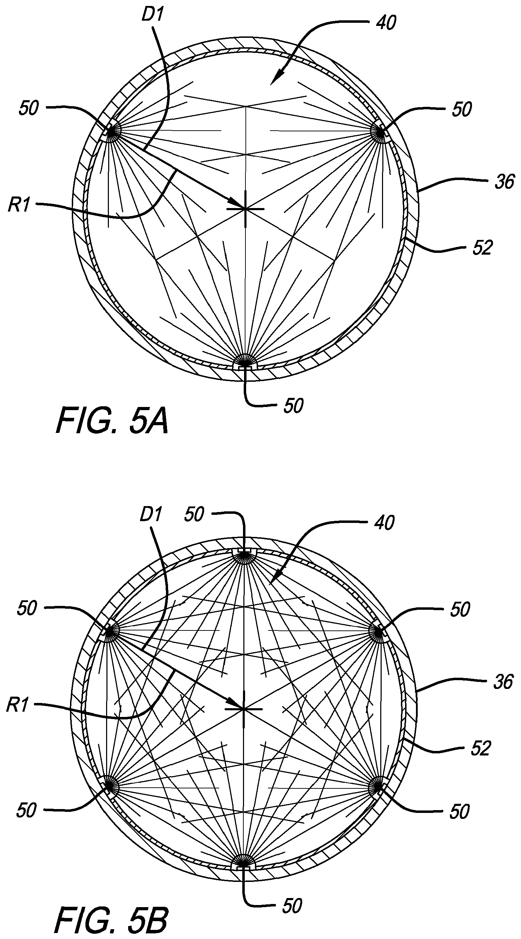

As shown in FIGS. 3 and 4, the sanitization assembly 30 includes a duct portion 36 that includes an inner surface 38 that defines a duct interior 40, an inlet 42, an outlet 44 and an airflow path (see arrows P1) that extends from the inlet 42, through the duct interior 40 and out the outlet 44. A fan 46 is positioned to move air in through the inlet 42 and out through the outlet 44. The fan 46 or other air moving component can be located in the duct interior or exterior to the duct portion. The sanitization assembly 30 includes an air sanitizing light system 48 that includes one or more sanitizing light emitters 50 disposed in the duct interior 40 between the inlet and the outlet. As described herein, the sanitizing light emitters 50 can be UV lights, LEDs or any type of light emitting device that emits light of a wavelength that sanitizes and kills or destroys pathogens and the like. A reflective coating 52 (see FIGS. 5A-5D) is disposed on at least a portion of the inner surface 38 of the duct.

As shown in FIG. 2, in a preferred embodiment, the interior/exterior sanitization assembly 30a is positioned between the sink 34 and the mirror 32. Preferably, the sink 34 includes a backsplash 54 and the interior/exterior sanitization assembly 30a is positioned between the top of the backsplash 54 and below the bottom of the mirror 32. The duct portion 36 of the interior/exterior sanitization assembly 30a includes a transparent section 56 defined therein. For example, the transparent section 56 can be a transparent cover or lens that is secured or positioned in an opening defined in the duct portion 36. It will be appreciated that the transparent section 56 allows some of the light emitted from the sanitizing light emitters 50 to pass through the transparent section, to the exterior of the duct portion or sanitization assembly so the light can sanitize components, air or the like outside or exterior to the sanitization assembly. As shown in FIG. 3, certain sanitizing light emitters 50 can be positioned such that light emitted therefrom is directed through the transparent section 56. These sanitizing light members 50 together may be referred to herein as an exterior sanitizing light system 58. As shown in FIG. 2, in a preferred embodiment, the inlet 42 and/or the fan 46 extends beyond or further outboard than the outboard side surfaces of the sink and mirror. A person of ordinary skill in the art will appreciate that the first wall of the lavatory monument assembly 10 is configured to be positioned against the wall of an aircraft (i.e., outboard from the second wall). The sanitization assemblies 30 preferably include a power source, such as a wired connection to electricity or a battery, for powering the lights, fan, etc.

In the embodiment shown in FIGS. 1 and 2, the light directed through the transparent section 56 is directed to the sink and surfaces thereon (e.g., faucet, counter, dispensers, sink bowl, etc.) to sanitize those surfaces. Therefore, the interior/exterior sanitization assembly 30a helps sanitize the air within the lavatory interior by moving the air through the duct portion and also helps sanitize the surfaces on or near the sink. It will be appreciated that the exterior sanitizing light emitters that direct light through the transparent section will also sanitize some of the air within the duct interior. The interior/exterior sanitization assembly 30a can also be positioned in other locations such it can sanitize other surfaces, such as above the toilet or the door handle.

In a preferred embodiment, the sanitization assembly 30 may also include a fragrance dispenser positioned within the airflow path at any point between the inlet 42 and the outlet 44. FIG. 3 shows a fragrance dispenser 60 embodied as a wicking fabric through which at least a portion of the air moving along the airflow path P1 moves. However, any type of fragrance dispenser or component for providing a scent to the exiting air is within the scope of the present invention.

As shown in FIG. 4, in a preferred embodiment, the interior sanitization assembly 30b includes a mounting member 62 that includes a main body portion 64 that may be connected or attached to a surface, such as a wall, and first and second bracket members 66 extending from and/or attached to the main body portion 64. The first and second bracket members 66 each include an opening 68 defined therein or therethrough and the duct portion 36 extends between and through the openings 68. A transparent member or section can also be added to the sanitizing assembly 30b shown in FIG. 4.

As shown in FIGS. 5A-5D, any or all of the sanitizing light emitters 50 (interior air or exterior sanitizing light members) are arranged in various patterns and/or one or more arrays around the interior or inner surface 38 of the duct portion 30. Preferably, the duct portion 30 also includes the UV reflective coating 52 on the inside of the duct in order to reflect the light waves in the duct interior 40 through which the air flows and the airflow path P1 extends so as to help provide enough energy to kill airborne pathogens in the desired or predetermined amount of time. For example, the air sanitizing light system 48 in any of the sanitization assemblies 30, 30a or 30b can include a plurality of sanitizing light emitters 50 arranged in a circumferential array about the inner surface of the duct portion, as shown in FIGS. 5A-5D. FIG. 4 shows the sanitizing light emitters 50 in a helix pattern or array. It will be appreciated that in an embodiment where the duct portion or the inner surface thereof is not circular, the sanitizing light emitters 50 can still be arranged circumferentially, meaning they are arranged around or thereabout the inner surface.

As shown in FIG. 5A, the inner surface 38 of the duct portion 36 defines a radius R1 that extends from the inner surface to the center of the circle in an embodiment, where the duct portion is circular or cylindrical. It will be appreciated that axis of the duct portion extends along the center of the duct portion. Therefore, in an embodiment, where the duct portion or the inner surface thereof is not circular, the radius or line R1 extends from the inner surface to the axis of the duct portion. The air sanitizing light emitters 50 within an array can be arranged to all direct light inwardly parallel to or along the radius R1 (or radially inwardly), as shown in FIGS. 5A and 5B. In FIGS. 5A and 5B, each light emitter 50 includes a plurality or set of lines extending therefrom representing the light or light rays shining or emitting from the emitter. The set of lines includes a center line or arrow labeled D1 that represents the angle that the direct light is emitting from the emitter. As shown in FIGS. 5A and 5B, the direct line D1 is coincident and/or parallel to the radius R1, showing that the light is directed along the radius R1 and toward the axis of the duct portion or duct interior.

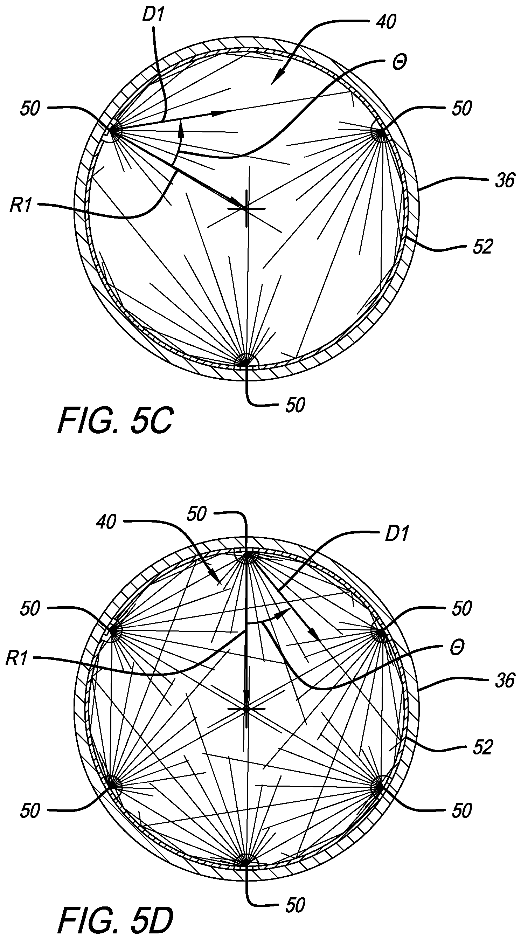

In another embodiment, the air sanitizing light emitters within an array can be arranged to all direct light at a non-zero angle θ (non-parallel or any angle between 1° and 89°) to the radius R1, as shown in FIGS. 5C and 5D. In other words, as shown in FIGS. 5C and 5D, the direct arrow or line D1 is angled with respect to the radius R1, showing that the light is directed at a non-zero angle θ (non-parallel) to the radius R1. Line D1 also is shown as reflecting off of the reflective coating 52 in FIGS. 5C and 5D. The reflective coating can coat the entire inner surface of the duct portion and can be positioned in an number of predetermined areas to strategically reflect the light from various emitters.

In another embodiment, some air sanitizing light emitters 50 can be arranged to direct light parallel with the radius and others within the same array can be arranged to direct light at a non-zero angle.

The above-detailed description of embodiments of the disclosure is not intended to be exhaustive or to limit the teachings to the precise form disclosed above. While specific embodiments of and examples for the disclosure are described above for illustrative purposes, various modifications are possible within the scope of the invention as defined by the claims.

## Claims

1. A sanitization assembly (30) comprising: a duct portion (36) that includes an inner surface (38) that defines a duct interior (40), an inlet (42), an outlet (44) and an airflow path that extends from the inlet (42), through the duct interior (40) and out the outlet (44), a fan (46) positioned to move air in through the inlet (42) and out through the outlet (44), an air sanitizing light system (48) that includes at least a first air sanitizing light emitter (50) disposed in the duct interior (40) between the inlet (42) and the outlet (44), and a reflective coating (52) is disposed on at least a portion of the inner surface (38), **characterized in that** the air sanitizing light system (48) includes a plurality of air sanitizing light emitters (50) arranged in a first circumferential array about the inner surface (38) of the duct portion (36), and wherein the duct portion (36) includes a transparent section (56) defined therein, wherein an exterior sanitizing light system (48) that includes at least a first exterior sanitizing light emitter (50) is disposed in the duct interior (40), wherein the first exterior sanitizing light emitter (50) is positioned to direct light through the transparent section (56) and to an exterior of the duct portion (36).

2. The sanitization assembly (30) of claim 1, wherein the air moving through the duct interior (40) cools the air sanitizing light emitters (50).

3. A vehicle lavatory monument assembly (10) comprising:
an enclosure (12) that includes a plurality of walls (14, 16, 18) that cooperate to define a lavatory interior (22), wherein an entry door (28) is positioned on one of the plurality of walls (14, 16, 18), and
a sanitization assembly (30) according to claim 1 or 2, positioned in the lavatory interior (22),
a sink (34) positioned in the lavatory interior (22), wherein the sanitization assembly (30) is positioned above the sink (34).

4. The vehicle lavatory monument assembly (10) of claim 3 further comprising a fragrance dispenser (60) positioned within the airflow path between the inlet (42) and the outlet (44).

5. The vehicle lavatory monument assembly (10) of claim 3 wherein the inner surface (38) of the duct portion (36) defines a radius (R1), wherein the air sanitizing light emitters (50) in the first circumferential array are arranged to direct light radially inwardly.

6. The vehicle lavatory monument assembly (10) of claim 3 wherein the air sanitizing light emitters (50) in the first circumferential array are arranged to direct light at a non-zero angle to the radius (R1).

7. The vehicle lavatory monument assembly (10) of claim 3 wherein the air sanitizing light emitters (50) in the first circumferential array are arranged in a helical pattern.

8. The vehicle lavatory monument assembly (10) of claim 3 wherein the sanitization assembly (30) includes a mounting member (62) that includes a main body portion (64) and first and second bracket members connected to the main body portion (64), wherein the first and second bracket members (66) each include an opening (68) defined therethrough, wherein the duct portion (36) extends through the openings (68) in the first and second bracket members (66).

9. The vehicle lavatory monument assembly (10) of claim 3 wherein the air moving through the duct interior (40) cools the first sanitizing light emitter (50).

10. The vehicle lavatory monument assembly (10) of claim 4 wherein the fragrance dispenser (60) is positioned within the airflow path between the inlet (42) and the transparent section (56).

11. The vehicle lavatory monument assembly (10) of claim 4 wherein the fragrance dispenser (60) is positioned within the airflow path and adjacent the inlet (42).

12. The vehicle lavatory monument assembly (10) of claim 4 wherein the fragrance dispenser (60) is positioned within the airflow path and adjacent the outlet (44).

13. A vehicle lavatory monument assembly (10) comprising:
an enclosure (12) that includes a plurality of walls (14, 16, 18) that cooperate to define a lavatory interior (22), wherein an entry door (28) is positioned on one of the plurality of walls (14, 16, 18), wherein a sink (34) is positioned on one of the plurality of walls (14, 16, 18), wherein the sink (34) includes a backsplash (54), wherein a mirror (32) is positioned above the sink (34), and
a first sanitization assembly (30) according to claim 1, positioned between the mirror (32) and the backsplash (54), wherein
the exterior sanitizing light system (48) includes a plurality of exterior sanitizing light emitters (50) disposed in the duct interior (40) and positioned to direct light through the transparent section (56) and to the sink (34),
a fragrance dispenser (60) positioned within the airflow path between the transparent section (56) and the outlet (44).

14. The vehicle lavatory monument assembly (10) of claim 13 further comprising a second sanitization assembly disposed in the lavatory interior (22), wherein the second sanitization assembly includes a duct portion (36) that includes an inner surface (38) that defines a duct interior (40), an inlet (42), an outlet (44) and an airflow path that extends from the inlet (42), through the duct interior (40) and out the outlet (44), wherein a reflective coating (52) is disposed on at least a portion of the inner surface (38), an air sanitizing light system (48) that includes a plurality of air sanitizing light emitters (50) arranged in a first circumferential array about the inner surface (38) of the duct portion (36) disposed in the duct interior (40) between the inlet (42) and the outlet (44), and a fragrance dispenser (60) positioned within the airflow path between the transparent section (56) and the outlet (44).

15. The vehicle lavatory monument assembly (10) of claim 14 wherein the sink (34) includes an outboard side surface, wherein the mirror (32) includes an outboard side surface, and wherein the outlet (44) and the fan (46) are positioned outboard of the outboard side surface.

## Patentansprüche

1. Desinfektionsbaugruppe (30), umfassend: einen Kanalabschnitt (36), der eine Innenfläche (38) einschließt, die einen Kanalinnenraum (40), einen Einlass (42), einen Auslass (44) und einen Luftstrompfad definiert, der sich von dem Einlass (42) durch den Kanalinnenraum (40) und aus dem Auslass (44) erstreckt, einen Lüfter (46), der so positioniert ist, dass er Luft durch den Einlass (42) herein und aus dem Auslass (44) heraus bewegt, ein Luftdesinfektionslichtsystem (48), das mindestens einen ersten Luftdesinfektionslichtstrahler (50), der in dem Kanalinnenraum (40) zwischen dem Einlass (42) und dem Auslass (44) angeordnet ist, einschließt, und wobei eine reflektierende Beschichtung (52) auf mindestens einem Abschnitt der Innenfläche (38) angeordnet ist, **dadurch gekennzeichnet, dass** das Luftdesinfektionslichtsystem (48) eine Vielzahl von Luftdesinfektionslichtstrahlern (50) umfasst, die in einer ersten Umfangsanordnung um die Innenfläche (38) des Kanalabschnitts (36) herum angeordnet sind, und wobei der Kanalabschnitt (36) einen darin definierten transparenten Abschnitt (56) einschließt, wobei ein äußeres Desinfektionslichtsystem (58), das mindestens einen ersten äußeren Desinfektionslichtstrahler einschließt, in dem Kanalinnenraum (40) angeordnet ist, wobei der erste äußere Desinfektionslichtstrahler so positioniert ist, dass er Licht durch den transparenten Abschnitt (56) und auf eine Außenseite des Kanalabschnitts (36) richtet.

2. Desinfektionsbaugruppe (30) nach Anspruch 1, wobei die sich durch den Kanalinnenraum (40) bewegende Luft die Luftdesinfektionslichtstrahler (50) kühlt.

3. Fahrzeugtoiletten-Monumentbaugruppe (10), umfassend:
eine Einhausung (12), die eine Vielzahl von Wänden (14, 16, 18) einschließt, die zusammenwirken, um einen Toiletteninnenraum (22) zu definieren, wobei eine Eingangstür (28) an einer der Vielzahl von Wänden (14, 16, 18) positioniert ist, und
eine Desinfektionsbaugruppe (30) nach Anspruch 1 oder 2, die in dem Toiletteninnenraum (22) positioniert ist,
ein Waschbecken (34), das in dem Toiletteninnenraum (22) positioniert ist, wobei die Desinfektionsbaugruppe (30) oberhalb des Waschbeckens (34) positioniert ist.

4. Fahrzeugtoiletten-Monumentbaugruppe (10) nach Anspruch 3, ferner umfassend einen Duftspender (60), der innerhalb des Luftstrompfads zwischen dem Einlass (42) und dem Auslass (44) positioniert ist.

5. Fahrzeugtoiletten-Monumentbaugruppe (10) nach Anspruch 3, wobei die Innenfläche (38) des Kanalabschnitts (36) einen Radius (R1) definiert, wobei die Luftdesinfektionslichtstrahler (50) in der ersten Umfangsanordnung angeordnet sind, um Licht radial nach innen zu richten.

6. Fahrzeugtoiletten-Monumentbaugruppe (10) nach Anspruch 3, wobei die Luftdesinfektionslichtstrahler (50) in der ersten Umfangsanordnung so angeordnet sind, dass sie Licht in einem Winkel, der nicht Null ist, zu dem Radius (R1) richten.

7. Fahrzeugtoiletten-Monumentbaugruppe (10) nach Anspruch 3, wobei die Luftdesinfektionslichtstrahler (50) in der ersten Umfangsanordnung in einem spiralförmigen Muster angeordnet sind.

8. Fahrzeugtoiletten-Denkmalbaugruppe (10) nach Anspruch 3, wobei die Desinfektionsbaugruppe (30) ein Befestigungselement (62) einschließt, das einen Hauptkörperabschnitt (64) und ein erstes und ein zweites Halterungselement umfasst, die mit dem Hauptkörperabschnitt (64) verbunden sind, wobei das erste und das zweite Halterungselement (66) jeweils eine durch sie hindurch definierte Öffnung (68) umfassen, wobei sich der Kanalabschnitt (36) durch die Öffnungen (68) in dem ersten und dem zweiten Halterungselement (66) erstreckt.

9. Fahrzeugtoiletten-Monumentbaugruppe (10) nach Anspruch 3, wobei die sich durch den Kanalinnenraum (40) bewegende Luft den ersten Desinfektionslichtstrahler (50) kühlt.

10. Fahrzeugtoiletten-Monumentbaugruppe (10) nach Anspruch 4, wobei der Duftspender (60) innerhalb des Luftstrompfads zwischen dem Einlass (42) und dem transparenten Abschnitt (56) positioniert ist.

11. Fahrzeugtoiletten-Monumentbaugruppe (10) nach Anspruch 4, wobei der Duftspender (60) innerhalb des Luftstrompfads und neben dem Einlass (42) positioniert ist.

12. Fahrzeugtoiletten-Monumentbaugruppe (10) nach Anspruch 4, wobei der Duftspender (60) innerhalb des Luftstrompfads und neben dem Auslass (44) positioniert ist.

13. Fahrzeugtoiletten-Monumentbaugruppe (10), umfassend:
eine Einhausung (12), die eine Vielzahl von Wänden (14, 16, 18) einschließt, die zusammenwirken, um einen Toiletteninnenraum (22) zu definieren, wobei eine Eingangstür (28) an einer der Vielzahl von Wänden (14, 16, 18) positioniert ist, wobei ein Waschbecken (34) an einer der Vielzahl von Wänden (14, 16, 18) positioniert ist, wobei das Waschbecken (34) einen Fliesenspiegel (54) einschließt, wobei ein Spiegel (32) oberhalb des Waschbeckens (34) positioniert ist, und
eine erste Desinfektionsbaugruppe (30) nach Anspruch 1, die zwischen dem Spiegel (32) und dem Fliesenspiegel (54) positioniert ist, wobei das äußere Desinfektionslichtsystem (58) eine Vielzahl von äußeren Desinfektionslichtstrahlern einschließt, die in dem Kanalinnenraum (40) angeordnet und so positioniert sind, dass sie Licht durch den transparenten Abschnitt (56) und zu dem Waschbecken (34) richten,
wobei ein Duftspender (60) innerhalb des Luftstrompfads zwischen dem transparenten Abschnitt (56) und dem Auslass (44) positioniert ist.

14. Fahrzeugtoiletten-Monumentbaugruppe (10) nach Anspruch 13, die ferner eine zweite Desinfektionsbaugruppe umfasst, die in dem Toiletteninnenraum (22) angeordnet ist, wobei die zweite Desinfektionsbaugruppe einen Kanalabschnitt (36), der eine Innenfläche (38) einschließt, die einen Kanalinnenraum (40), einen Einlass (42), einen Auslass (44) und einen Luftstrompfad definiert, der sich von dem Einlass (42) durch den Kanalinnenraum (40) und aus dem Auslass (44) erstreckt, wobei eine reflektierende Beschichtung (52) auf mindestens einem Abschnitt der Innenfläche (38) angeordnet ist, ein Luftdesinfektionslichtsystem (48), das eine Vielzahl von Luftdesinfektionslichtstrahlern (50) einschließt, die in einer ersten Umfangsanordnung um die Innenfläche (38) des Kanalabschnitts (36) herum angeordnet sind, der in dem Kanalinnenraum (40) zwischen dem Einlass (42) und dem Auslass (44) angeordnet ist, und einen Duftspender (60) einschließt, der innerhalb des Luftstrompfads zwischen dem transparenten Abschnitt (56) und dem Auslass (44) positioniert ist.

15. Fahrzeugtoiletten-Monumentbaugruppe (10) nach Anspruch 14, wobei das Waschbecken (34) eine Außenseitenfläche einschließt, wobei der Spiegel (32) eine Außenseitenfläche einschließt, und wobei der Auslass (44) und der Lüfter (46) außerhalb der Außenseitenfläche positioniert sind.

## Revendications

1. Ensemble d'assainissement (30) comprenant : une partie de conduit (36) qui inclut une surface interne (38) qui définit un espace intérieur de conduit (40), une entrée (42), une sortie (44) et un trajet de flux d'air qui s'étend depuis l'entrée (42), à travers l'espace intérieur de conduit (40) et hors de la sortie (44), un ventilateur (46) positionné pour faire entrer l'air à travers l'entrée (42) et le faire sortir à travers la sortie (44), un système de lumière d'assainissement d'air (48) qui inclut au moins un premier dispositif d'émission de lumière d'assainissement d'air (50) disposé dans l'espace intérieur de conduit (40) entre l'entrée (42) et la sortie (44), et un revêtement réfléchissant (52) est disposé sur au moins une partie de la surface interne (38), **caractérisé en ce que** le système de lumière d'assainissement d'air (48) inclut une pluralité de dispositifs d'émission de lumière d'assainissement d'air (50) agencés en un premier réseau circonférentiel autour de la surface interne (38) de la partie de conduit (36), et dans lequel la partie de conduit (36) inclut une section transparente (56) définie dans celui-ci, dans lequel un système de lumière d'assainissement extérieur (58) qui inclut au moins un premier dispositif d'émission de lumière d'assainissement extérieur est disposé dans l'espace intérieur de conduit (40), dans lequel le premier dispositif d'émission de lumière d'assainissement extérieur est positionné pour diriger la lumière à travers la section transparente (56) et vers un extérieur de la partie de conduit (36).

2. Ensemble d'assainissement (30) selon la revendication 1, dans lequel l'air circulant à travers l'espace intérieur de conduit (40) refroidit les dispositifs d'émission de lumière d'assainissement d'air (50).

3. Ensemble structure de toilettes (10) pour véhicule comprenant :
une enceinte (12) qui inclut une pluralité de parois (14, 16, 18) qui coopèrent pour définir un espace intérieur de toilettes (22), dans lequel une porte d'accès (28) est positionnée sur l'une de la pluralité de parois (14, 16, 18), et
un ensemble d'assainissement (30) selon la revendication 1 ou 2, positionné dans l'espace intérieur de toilettes (22),
un lavabo (34) positionné dans l'espace intérieur de toilettes (22), dans lequel l'ensemble d'assainissement (30) est positionné au-dessus du lavabo (34).

4. Ensemble structure de toilettes (10) pour véhicule selon la revendication 3, comprenant en outre un diffuseur de parfum (60) positionné dans le trajet de flux d'air entre l'entrée (42) et la sortie (44).

5. Ensemble structure de toilettes (10) pour véhicule selon la revendication 3, dans lequel la surface interne (38) de la partie de conduit (36) définit un rayon (R1), dans lequel les dispositifs d'émission de lumière d'assainissement d'air (50) dans le premier réseau circonférentiel sont agencés pour diriger la lumière radialement vers l'intérieur.

6. Ensemble structure de toilettes (10) pour véhicule selon la revendication 3, dans lequel les dispositifs d'émission de lumière d'assainissement d'air (50) dans le premier réseau circonférentiel sont agencés pour diriger la lumière selon un angle non nul par rapport au rayon (R1).

7. Ensemble structure de toilettes (10) pour véhicule selon la revendication 3, dans lequel les dispositifs d'émission de lumière d'assainissement d'air (50) dans le premier réseau circonférentiel sont agencés en un motif hélicoïdal.

8. Ensemble structure de toilettes (10) pour véhicule selon la revendication 3, dans lequel l'ensemble d'assainissement (30) inclut un organe de montage (62) qui inclut une partie de corps principal (64) et des premier et second organes de support reliés à la partie de corps principal (64), dans lequel les premier et second organes de support (66) incluent chacun une ouverture (68) définie à travers ceux-ci, dans lequel la partie de conduit (36) s'étend à travers les ouvertures (68) dans les premier et second organes de support (66).

9. Ensemble structure de toilettes (10) pour véhicule selon la revendication 3, dans lequel l'air circulant à travers l'espace intérieur de conduit (40) refroidit le premier dispositif d'émission de lumière d'assainissement (50).

10. Ensemble structure de toilettes (10) pour véhicule selon la revendication 4, dans lequel le diffuseur de parfum (60) est positionné dans le trajet de flux d'air entre l'entrée (42) et la section transparente (56).

11. Ensemble structure de toilettes (10) pour véhicule selon la revendication 4, dans lequel le diffuseur de parfum (60) est positionné dans le trajet de flux d'air et de manière adjacente à l'entrée (42).

12. Ensemble structure de toilettes (10) pour véhicule selon la revendication 4, dans lequel le diffuseur de parfum (60) est positionné dans le trajet de flux d'air et de manière adjacente à la sortie (44).

13. Ensemble structure de toilettes (10) pour véhicule comprenant :
une enceinte (12) qui inclut une pluralité de parois (14, 16, 18) qui coopèrent pour définir un espace intérieur de toilette (22), dans lequel une porte d'accès (28) est positionnée sur l'une de la pluralité de parois (14, 16, 18), dans lequel un lavabo (34) est positionné sur l'une de la pluralité de parois (14, 16, 18), dans lequel le lavabo (34) inclut une crédence (54), dans lequel un miroir (32) est positionné au-dessus du lavabo (34), et
un premier ensemble d'assainissement (30) selon la revendication 1, positionné entre le miroir (32) et la crédence (54), dans lequel le système de lumière d'assainissement extérieur (58) inclut une pluralité de dispositifs d'émission de lumière d'assainissement extérieurs disposés dans l'espace intérieur de conduit (40) et positionnés pour diriger la lumière à travers la section transparente (56) et vers le lavabo (34),
un diffuseur de parfum (60) positionné dans le trajet de flux d'air entre la section transparente (56) et la sortie (44).

14. Ensemble structure de toilettes (10) pour véhicule selon la revendication 13 comprenant en outre un second ensemble d'assainissement disposé dans l'espace intérieur de toilette (22), dans lequel le second ensemble d'assainissement inclut une partie de conduit (36) qui inclut une surface interne (38) qui définit un espace intérieur de conduit (40), une entrée (42), une sortie (44) et un trajet de flux d'air qui s'étend depuis l'entrée (42), à travers l'espace intérieur de conduit (40) et hors de la sortie (44), dans lequel un revêtement réfléchissant (52) est disposé sur au moins une partie de la surface interne (38), un système de lumière d'assainissement d'air (48) qui inclut une pluralité de dispositifs d'émission de lumière d'assainissement d'air (50) agencés en un premier réseau circonférentiel autour de la surface interne (38) de la partie de conduit (36) disposés dans l'espace intérieur de conduit (40) entre l'entrée (42) et la sortie (44), et un diffuseur de parfum (60) positionné dans le trajet de flux d'air entre la section transparente (56) et la sortie (44).

15. Ensemble structure de toilettes (10) pour véhicule selon la revendication 14, dans lequel le lavabo (34) inclut une surface latérale externe, dans lequel le miroir (32) inclut une surface latérale externe, et dans lequel la sortie (44) et le ventilateur (46) sont positionnés en périphérie de la surface latérale externe.
